# EUROPEAN PATENT SPECIFICATION

(11) **EP 2 344 884 B1**
(45) Date of publication and mention of the grant of the patent: **31.07.2013**
(21) Application number: 09819485.5
(22) Date of filing: 06.10.2009
(51) Int. Cl.: G01N 33/53, G01N 33/538, G01N 33/543, G01N 33/577

(54) **SEMI-SEQUENTIAL ASSAY FOR DETECTION OF AN ANALYTE IN A SAMPLE**
SEMISEQUENTIELLER TEST FÜR DEN NACHWEIS EINES ANALYTEN IN EINER PROBE
DOSAGE SEMI-SÉQUENTIEL POUR LA DÉTECTION D'UN ANALYTE DANS UN ÉCHANTILLON

(30) Priority: 07.10.2008 SE 0802108
(43) Date of publication of application: 20.07.2011
(73) Proprietor: GYROS PATENT AB, 751 83 Uppsala (SE)
(72) Inventor: INGANÄS, Mats, S-757 55 Uppsala (SE); ÖSTERLUND, Karolina, S-756 51 Uppsala (SE); HALJE, Anna, 752 28 Uppsala (SE)
(74) Representative: Widén, Björn
(86) International application number: PCT/SE2009/051107
(87) International publication number: WO 2010/042031

(56) References cited:
- WO-A1-2006/075965
- WO-A2-03/021222
- WO-A2-2005/108989
- WO-A2-2006/066912
- US-A- 4 098 876
- US-A1- 2007 231 829
- HOESEL W. ET AL: 'Development and evaluation of a new ELISA for the detection and quantification of antierythropoietin antibodies in human sera' JOURNAL OF IMMUNOLOGICAL METHODS vol. 294, no. 1-2, 2004, pages 101 - 110, XP004679759
- GROSS J. ET AL: 'Detection of anti-EPO antibodies in human sera by a bridging ELISA is much more sensitive when coating biotinylated rhEPO to streptavidin rather than using direct coating of rhEPO' JOURNAL OF IMMUNOLOGICAL METHODS vol. 313, no. L-2, 2006, pages 176 - 182, XP025158155
- BOURDAGE J.S. ET AL: 'An Affinity Capture Elution (ACE) assay for detection of anti-drug antibody to monoclonal antibody therapeutics in the presence of high levels of drug' JOURNAL OF IMMUNOLOGICAL METHODS vol. 327, no. 1-2, 2007, pages 10 - 17, XP022266386

## Description

### Field of the Invention

The present invention relates to a method for detection of an analyte in a sample. The method is a semi-sequential assay procedure. The present invention further relates to a device for detection of an analyte in a sample.

### Background of the Invention

A key issue for the successful implementation of therapeutic regimens based on biotherapeutics is to minimize the patient immune response against the drug molecule. A number of factors may contribute to undesired immune responses, e.g. the generation of anti-drug antibodies (ADAs) against the drug molecule (DM):
- Immune response against drug molecules that contain portions of foreign proteins
- Mechanisms breaking immune tolerance against drug molecule
- Purity of the DM
- Glycosylation of the DM
- Aggregation of the DM
- Formulation of the DM
- Route of administration of the DM
- Immune status of patient

When the immune tolerance against the DM is broken, the appearance of anti-drug antibodies (ADAs) may have several types of unwanted effects:
- Increased elimination of the DM from circulation (i.e. reduced half-life of the DM in the patient)
- Loss of therapeutic efficacy
- Increased risk for adverse reactions. This may include anaphylactic as well as less severe reactions prohibiting further treatment of drug.
- Anti-drug antibodies (ADAs) may react with endogenously produced protein eliminating completely the supply of DM.

The formation of anti-drug antibodies upon treatment with recombinant proteins have been reviewed extensively (Koren E, Zuckerman LA, Mire-Sluis A. Immune responses to therapeutic proteins in humans- Clinical significance, assessment and prediction. Current Pharmaceutical Biotechnology; 2002, 3, 349-360; Pendley C, Schantz A, Wagner C. Immunogenicity of therapeutic monoclonal antibodies. Current opinion in Molecular Therapeutics; 2003, 5, 172-179; Shankar G, Pendley C, Stein KE. A risk-based bioanalytical strategy for the assessment of antibody immune responses against biological drugs. Nat. Biotechnology; 2007, 25, 555-561).

In order to understand the immunogenic potential of a candidate DM the development of anti-drug antibodies (ADAs) is monitored during clinical studies. This is performed according to a well developed process involving screening assays, confirmatory assays and assays for characterization of immune response following guidelines from regulatory authorities. There are explicit recommendations on how these procedures shall be conducted (Koren E, Smith HW, Shores E, Shankar G, Finco-Kent D, Rup B, Barrett Y-C, Devanarayan V, Gorowits B, Gupta S, Parish T, Quarmby V, Moxness M, Swanson, SJ, Taniguchi G, Zuckerman LA, Stebbins CC, Mire-Sluis A. Recommendations on risk-based strategies for detection and characterization of antibodies directed against biotechnology products. J. Immunol. Methods; 2008, 333, 1-9).

Depending on the nature of a Drug Molecule (DM) and the development of anti-drug antibodies (ADAs), the assay design(s) and technologies employed for accurate detection of anti-drug antibodies (ADAs) may differ (Mire-Sluis AR, Barrett YC, Devanarayan V, Koren E, Liu H, Maia M, Parish T, Scott G, Shankar G, Shores E, Swanson SJ, Taniguchi G, Wierda D, Zuckerman L. J. Immunol. Methods; 2004, 289, 1-16; Lofgren JA, Dhandapani S, Pennucci JJ, Abbott CM, Mytych DT, Kaliyaperumal A, Swanson SJ, Mullenix MC. Comparing ELISA and surface plasmon resonance for assessing clinical immunogenicity of panitumumab. J. Immunol. 2007, 178, 7467-7472).

The known procedures for detecting analytes, such as an anti-drug antibodies, in a sample, suffers from problems such as low signal to noise ratios, low sensitivity, and prozone effects (or hook effects). WO 2006/066912 and Hoesel et al., J. Imm. Meth. 294 (2004), 101-110 disclose methods of detecting therapeutic antibodies and ant-erythropoietin antibodies, respectively.

### Summary of the invention

In view of the problems associated with prior art assay methods for detection of an analyte such as an anti- drug antibody, in a sample, *i.e*. low signal to noise ratios, low sensitivity, and prozone effects (or hook effects), it is the object of the present invention to provide a method for detection of an analyte in a sample wherein the mentioned problems are eliminated or at least alleviated.

This object is met by a method for detection of an analyte in a sample, wherein the method comprises the following steps:
a) a saturating amount of a capture reagent, wherein the capture reagent comprises a capturing moiety and an affinity counterpart to the analyte, is contacted with a substrate having a surface with reactive sites capable of binding to the capturing moiety; and
b) the capture reagent binds to the substrate via the capturing moiety and is thereby immobilized, said reactive sites being saturated with the immobilized capture reagent; and
c) the sample comprising the analyte is contacted with a detection reagent, wherein the detection reagent comprises a detectable moiety and an affinity counterpart to the analyte; and
d) the analyte binds to the detection reagent via the affinity counterpart to form a complex; and
e) the complex is contacted with the capture reagent immobilized on the substrate, so that the complex binds via the analyte to the capture reagent immobilized on the substrate; and
f) the detectable moiety is detected,
wherein the analyte is an anti-drug antibody, the capture reagent comprises a drug molecule linked to the capturing moiety, and the detection reagent comprises a drug molecule linked to the detectable moiety, characterized in that the substrate is provided in a column and the drug molecule is an antibody.

In a further preferred embodiment the invention provides a method wherein the analyte is a drug molecule, the capture reagent comprises an anti-idiotypic antibody to the drug molecule linked to the capturing moiety, and the detection reagent comprises an anti-idiotypic antibody to the drug molecule linked to the detectable moiety.

In a further preferred embodiment the invention provides a method further comprising a step of washing the substrate with a washing solution prior to step f).

In a further preferred embodiment the invention provides a method wherein the washing solution comprises a detergent at a concentration level less than 5%, preferably less than 1%, more preferably less than 0.5%.

In a further preferred embodiment the invention provides a method wherein the detergent is non-ionic.

In a further preferred embodiment the invention provides a method wherein the detergent is polyoxyethylenesorbitan monolaurate.

In a further preferred embodiment the invention provides a method wherein the sample is contacted with the detection reagent for a time less than 24 hours prior to step e), preferably less than 2 hours prior to step e), more preferably less than 20 minutes prior to step e), more preferably less than 10 minutes prior to step e), more preferably less than 1 minute prior to step e), more preferably less than 1 second prior to step e).

In a further preferred embodiment the invention provides a method wherein the capturing moiety is biotin and the substrate having the surface with reactive sites comprises streptavidin.

In a further preferred embodiment the invention provides a method wherein the detectable moiety (3b) is selected from a group of reagents consisting of: Alexa Fluor 633, Alexa Fluor 647, DyLight 649, Cy-5, Cy-5.5, and fluorescent nanoparticles.

In a further preferred embodiment the invention provides a method wherein the sample has a volume less than, or equal to, 1000 nl, preferably less than, or equal to, 200 nl.

In a further preferred embodiment the invention provides a method wherein the detection reagent (3) has a volume less than, or equal to, 1000 nl, preferably less than, or equal to, 200 nl.

In a further preferred embodiment the invention provides a method wherein the detection reagent (3) has a concentration less than 15 µg/ml, or less than 100 nM, preferably less than 0.5 µg/ml, or less than 3.3 nM.

In a further preferred embodiment the invention provides a method wherein the substrate (4) comprises particles that are porous, or semi-pellicular, or pellicular (i.e. non porous).

In a further preferred embodiment the invention provides a method wherein the particles are essentially circular and have an average diameter higher than 10 µm.

In a further preferred embodiment the invention provides a method wherein the particles have a homogenous size distribution.

In a further preferred embodiment the invention provides a method wherein the substrate is a part of a device, wherein the device comprises:
- a sample inlet; and
- a detection reagent inlet; and
- a capture reagent inlet; and
- a mixing chamber in fluid contact with the sample inlet and the detection reagent inlet, wherein the mixing chamber is positioned in a direction downstream of the sample inlet and the detection reagent inlet; and
- a column comprising the substrate, wherein the column is in fluid contact with the sample inlet, the detection reagent inlet, the capture reagent inlet, and the mixing chamber, and wherein the column is positioned in a direction downstream of the capture reagent inlet and the mixing chamber; and
- an outlet in fluid contact with the column, wherein the outlet is positioned in a direction downstream of the column; and
- a means for detecting the detection reagent.

In another aspect of the present disclosure there is provided a device comprising:
- a sample inlet; and
- a detection reagent inlet; and
- a capture reagent inlet; and
- a mixing chamber in fluid contact with the sample inlet and the detection reagent inlet, wherein the mixing chamber is positioned in a direction downstream of the sample inlet and the detection reagent inlet; and
- a column comprising a substrate, wherein the substrate has a surface with reactive sites capable of binding to a capture reagent via a capturing moiety, wherein the column is in fluid contact with the sample inlet, the detection reagent inlet, the capture reagent inlet, and the mixing chamber, and wherein the column is positioned in a direction downstream of the capture reagent inlet and the mixing chamber; and
- an outlet in fluid contact with the column, wherein the outlet is positioned in a direction downstream of the column; and
- a means for detecting the detection reagent.

In a preferred embodiment of the disclosure of the device the reactive sites have been saturated with the capture reagent.

In a further preferred embodiment the disclosure provides a device wherein the capture reagent comprises a drug molecule linked to the capturing moiety.

In a further preferred embodiment the disclosure provides a device wherein the capture reagent comprises an anti-idiotypic antibody to a drug molecule linked to the capturing moiety.

In a further preferred embodiment the disclosure provides a device wherein the capturing moiety is biotin and the substrate having the surface with reactive sites comprises streptavidin.

In a further preferred embodiment the disclosure provides a device wherein the column has capacity to bind up to 1 pmole of the capture reagent, and wherein the column has a volume less than 20 nl.

In a further preferred embodiment the disclosure provides a device adapted to propagate the sample and the capture reagent at a flow rate in a range 0.1 to 5 nl/s, preferably in the range 0.3 to 2 nl/s.

### Brief Description of the Drawing

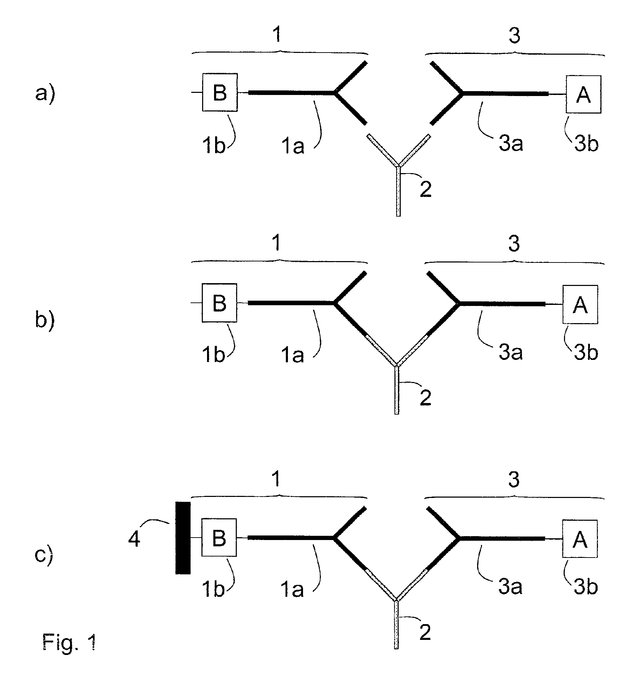

Fig. 1 illustrates a homogenous assay procedure wherein a) capture reagent (1), analyte (2), and detection reagent (3) is premixed; and b) the capture reagent (1), analyte (2), and detection reagent (3) forms a complex; and c) the complex is captured on a substrate (4).

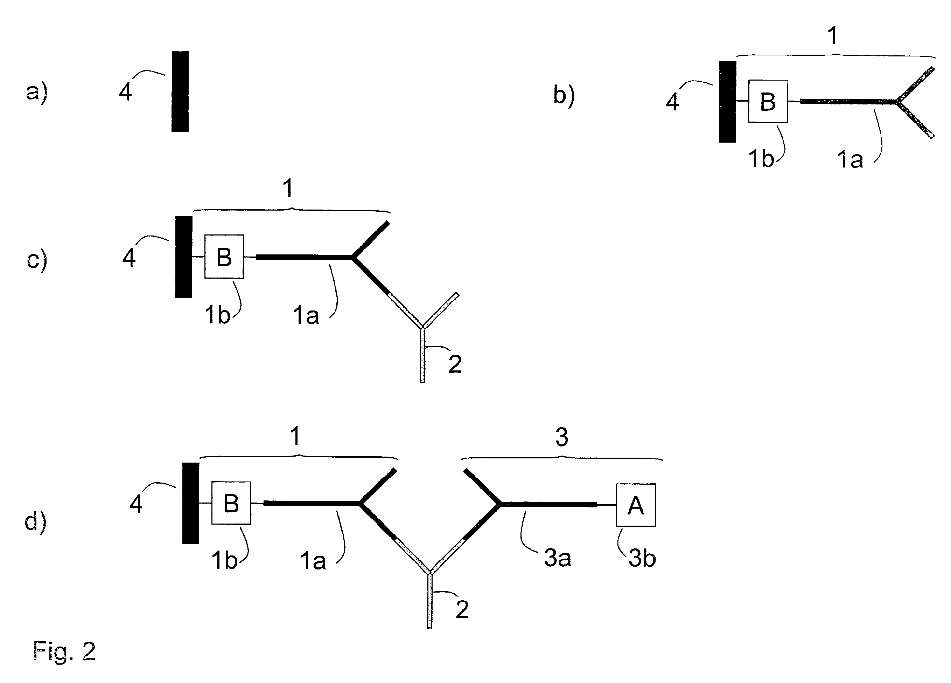

Fig. 2 illustrates a sequential assay procedure wherein a) a substrate (4) is used to b) bind a capture reagent (1) added thereto, in the next step c) an analyte (2) is added, and the analyte (2) binds to the capture reagent (1) immobilized on the substrate (4), in a further step d) a detection reagent (3) is added, and the detection reagent (3) binds to the analyte (2) that has previously been bound to the capture reagent (1) immobilized on the substrate (4).

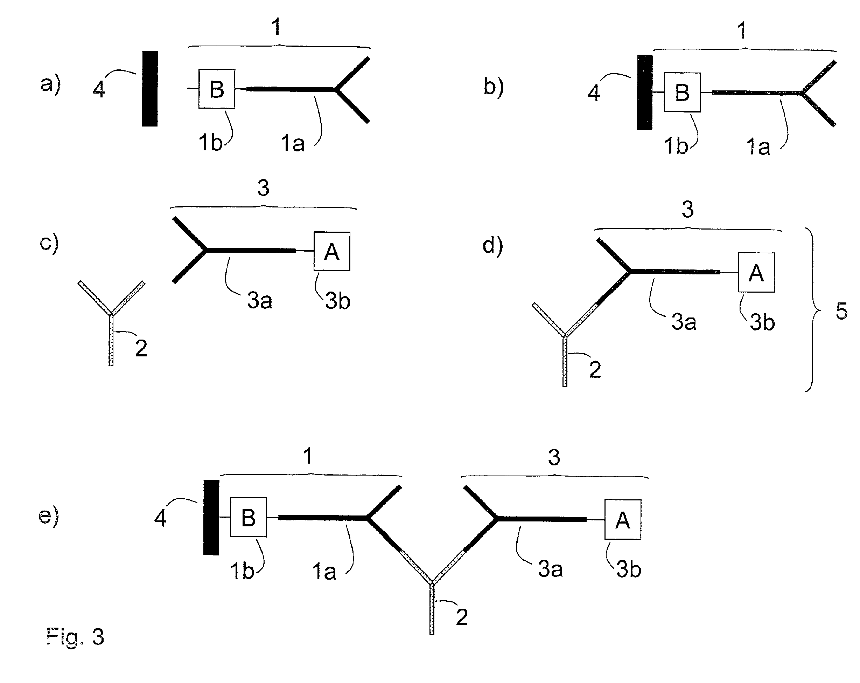

Fig. 3 illustrates a semi-sequential assay procedure wherein a) a capture reagent (1) is added to a substrate (4); and b) the capture reagent (1) binds to the substrate (4) and is thereby immobilized; and c) an analyte (2) is mixed with a detection reagent (3); and d) the analyte (2) binds to the detection reagent (3) to form a complex (5); and e) the complex (5) is added to the capture reagent (1) immobilized on the substrate (4), so that the complex (5) binds to the capture reagent (1) immobilized on the substrate (4).

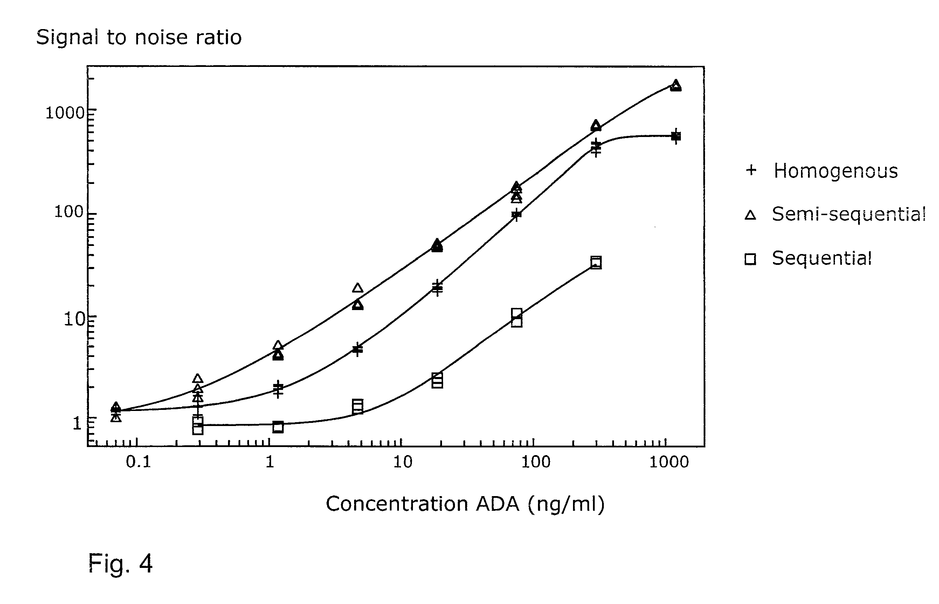

Fig. 4 illustrates a dose-response curves for the different assay protocols (homogenous procedure, sequential procedure, and semi-sequential procedure) using the same set up of immunoreagents.

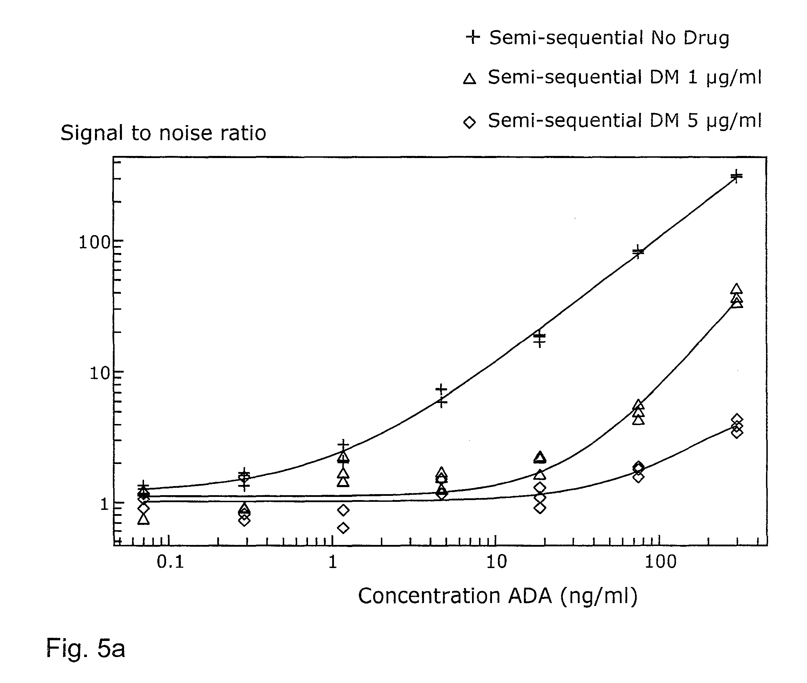

Fig. 5a) illustrates evaluation of drug tolerance in a semi-sequential assay procedure. In order to evaluate the effect of pre-existing DM (*i.e.* DM that is not linked to a capturing moiety or to a detectable moiety) upon detection of ADA, ADA at varying concentrations and Alexa labelled DM was premixed and analysed with three different levels of DM (0 µg/ml, 1 µg/ml, and 5 µg/ml) added to the sample.

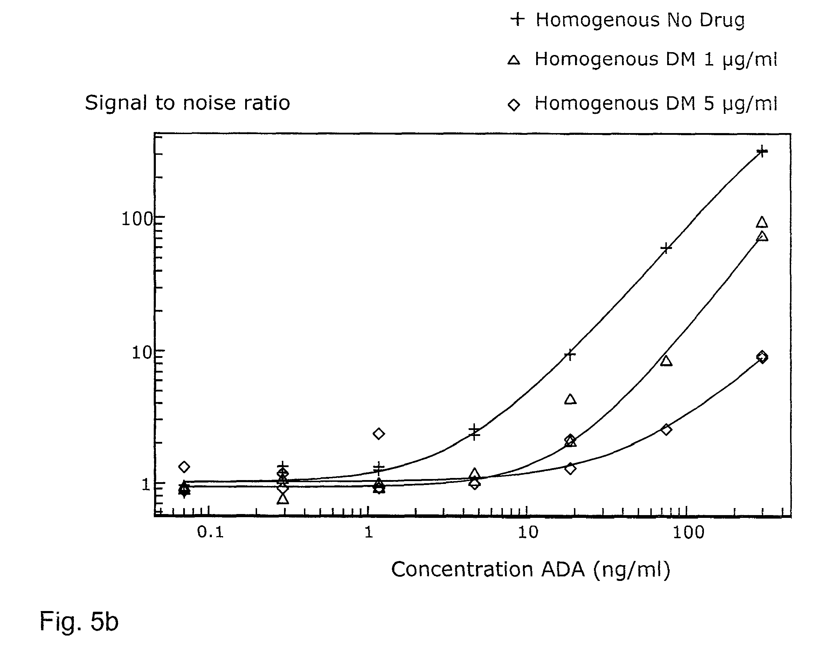

Fig. 5b) illustrates evaluation of drug tolerance in a homogenous assay procedure. In order to evaluate the effect of pre-existing DM (*i.e.* DM that is not linked to a capturing moiety or to a detectable moiety) upon detection of ADA, ADA at varying concentrations and Alexa labelled DM was premixed and analysed with three different levels of DM (0 µg/ml, 1 µg/ml, and 5 µg/ml) added to the sample.

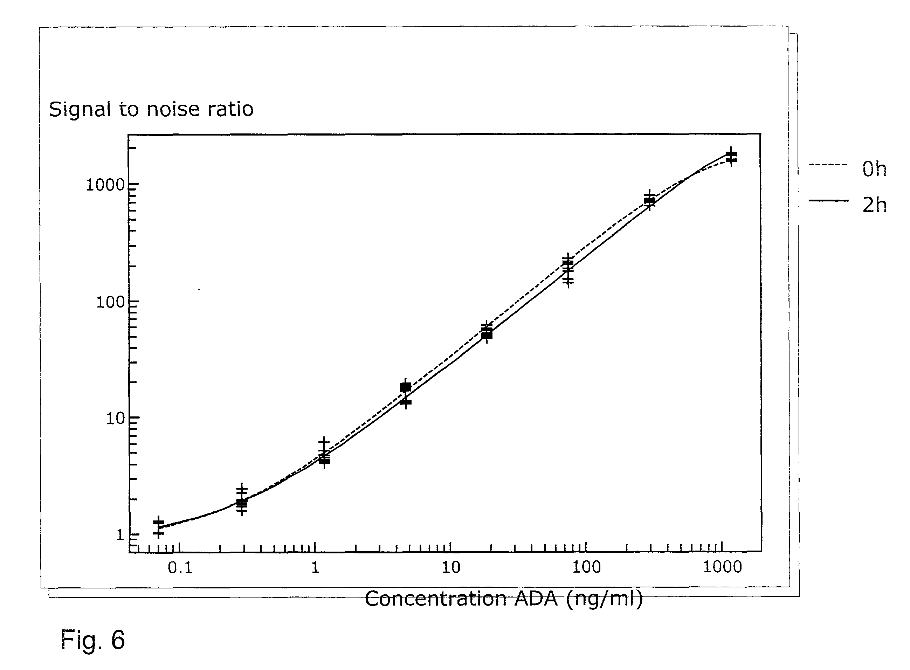

Fig. 6 illustrates effect of time for pre-incubation of ADA and Alexa-labelled DM in semi-sequential format.

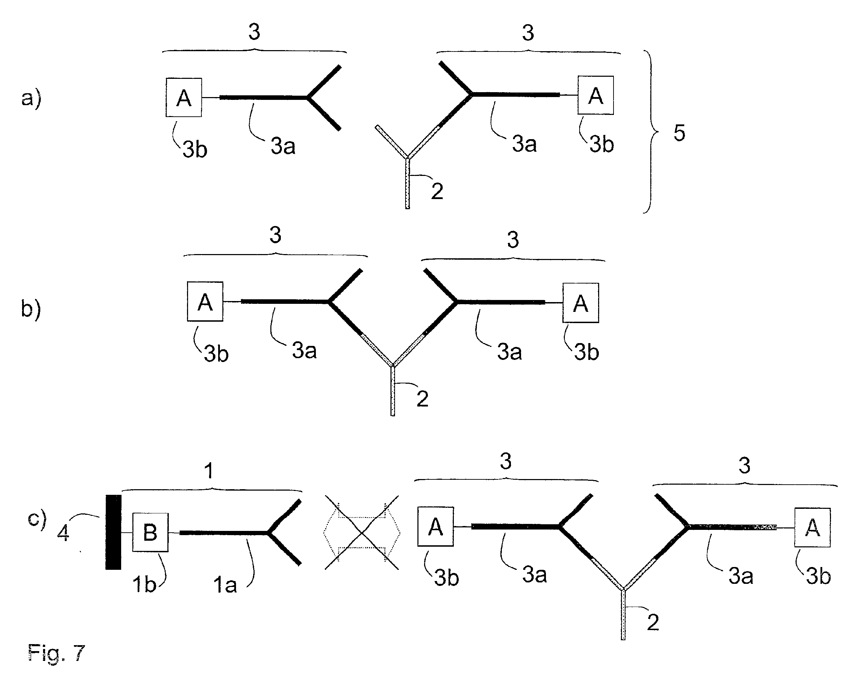

Fig. 7a)-c) illustrates a process, wherein a complex (5) has been formed between the analyte (2) and the detection reagent (3), if there is an excess of the detection reagent (3) compared to analyte (2), and/or if the mixing time for mixing the analyte (2) and the detection reagent (3) is relatively long, the complex (5) may bind to another unit of the detection reagent (3) to form a complex comprising two units of the detection reagent (3) and one unit of the analyte (2) (Fig. 7b)), and, as shown in c), such a complex will not bind to a capture reagent (1) immobilized on a substrate (4).

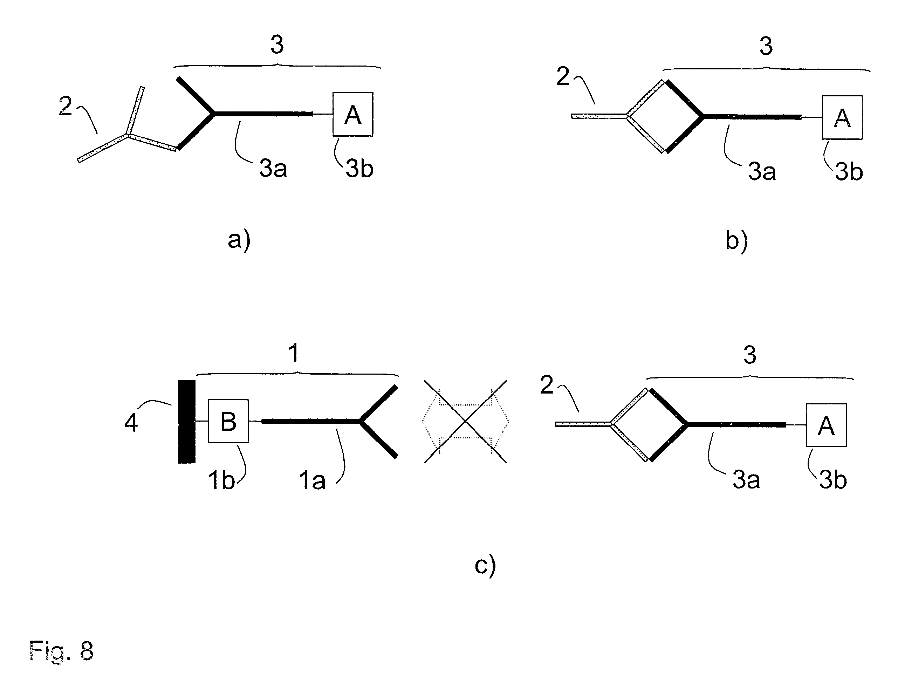

Fig. 8a) illustrates a process, wherein a bivalent analyte (2) binds to a detection reagent (3) via one of the two binding sites available on the analyte (2).
Fig. 8b) illustrates a process, wherein the bivalent analyte (2) bound to the detection reagent (3) in Fig. 6a) is given time to proceed with the binding process, so that it binds with both of the two binding sites to the detection reagent (3).
Fig. 8c) illustrates that the bivalent analyte (2) bound to the detection reagent (3) via both of the two binding sites will be unable to bind to a capture reagent (1) immobilized on a substrate (4).

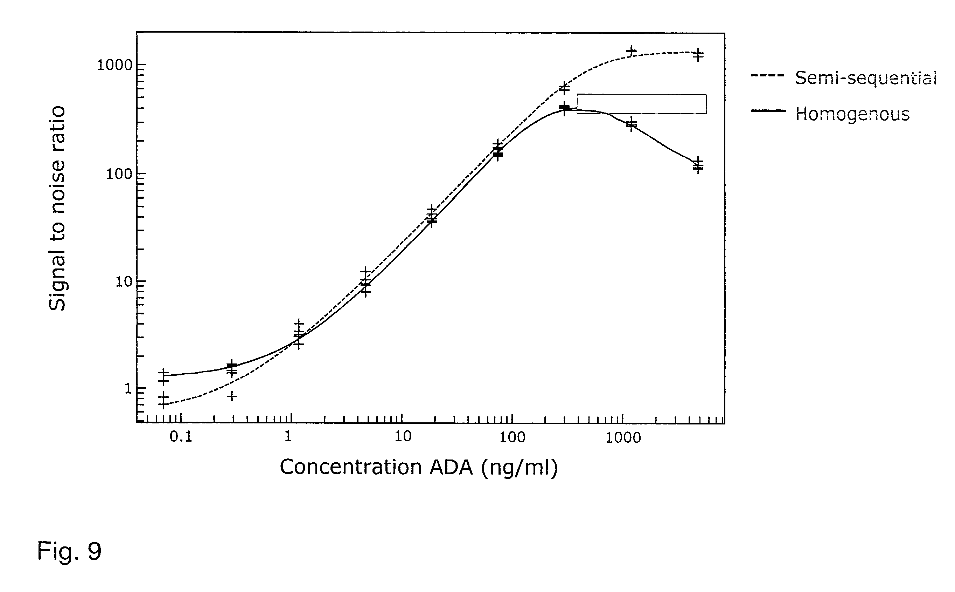

Fig. 9 illustrates sensitivity for the hook effect in the upper end of the concentration range for the homogeneous and semi-sequential formats, respectively.

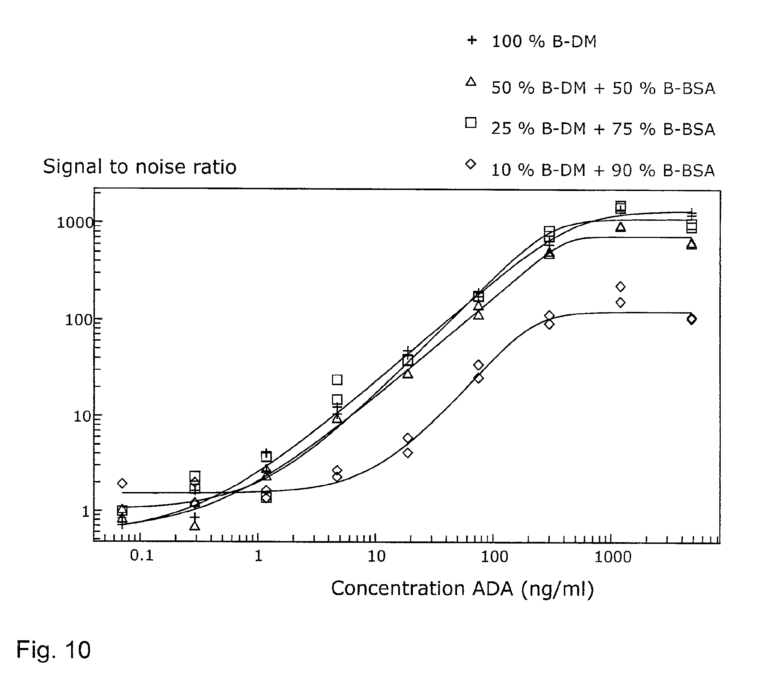

Fig. 10 illustrates dependence of capture column capacity for assay performance in the semi-sequential format. The figures in the panel represent the proportions of B-DM and B-BSA (100, 50/50, 25/75 and 10/90 %, respectively) on the capture column.

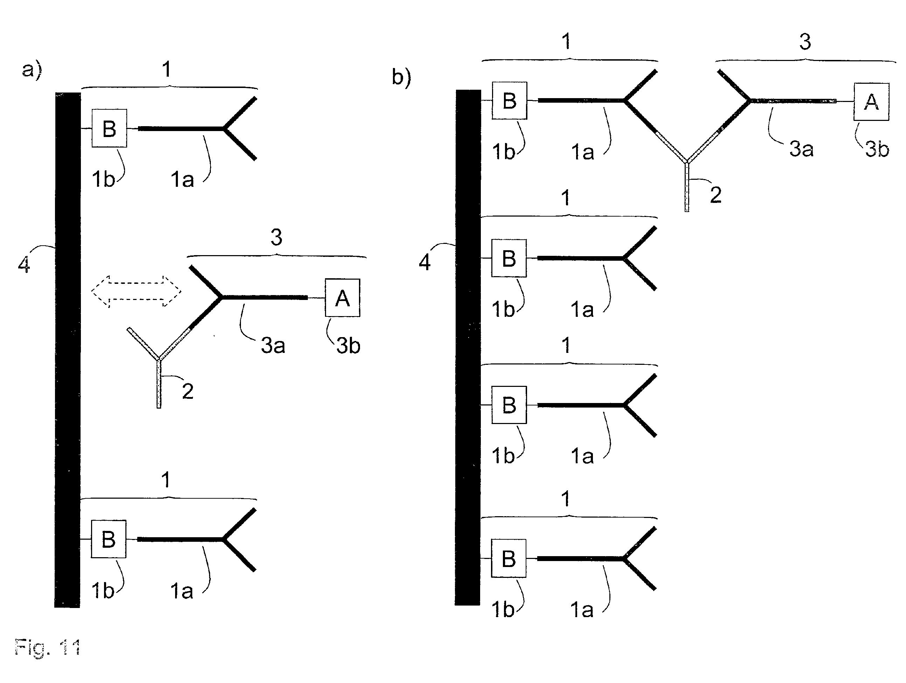

Fig. 11a) illustrates a process, wherein a substrate (4) is binding two units of a capture reagent (1), the substrate (4) is not saturated with capture reagent (1), therefore a part of the surface of substrate (4) is not covered with the capture reagent (1). As illustrated by the arrow, a complex formed between the analyte (2) and the detection reagent (3) can interact with the substrate (4) and even bind non-specifically to the substrate (4) and thereby increase the background signal in the system.
Fig. 11b) illustrates a process, wherein a substrate (4) has been contacted with a saturating solution of the capture reagent (1), so that the distance between the capture reagent (1) units immobilized on the substrate (4) is minimized. Thereby the surface of the substrate (4) available for non-specific interaction with, for example, the complex formed between the analyte (2) and the detection reagent (3), is minimized.

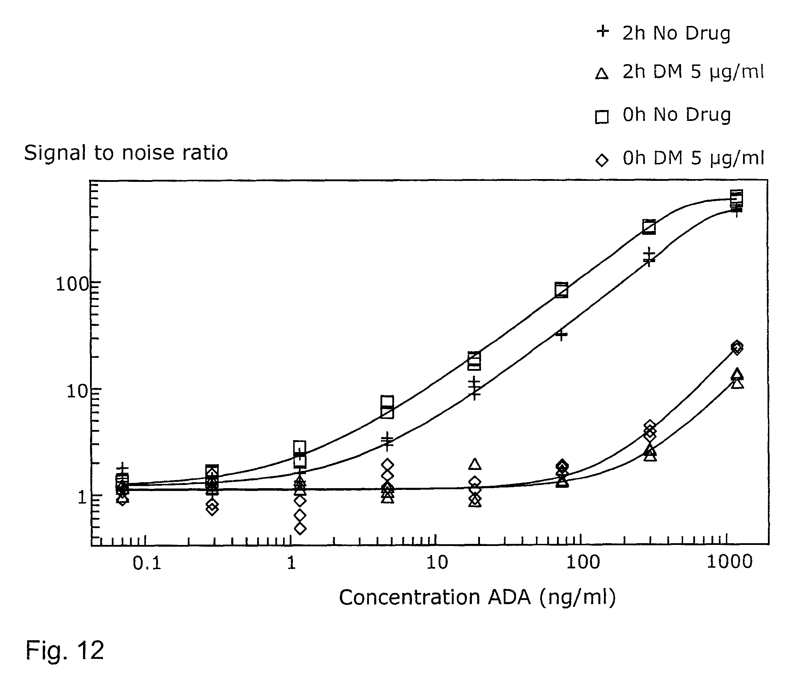

Fig. 12 illustrates evaluation of drug tolerance and the influence of incubation time in a semi-sequential assay procedure. In order to evaluate the time requirements for pre-incubation of ADA and Alexa labelled DM (DM-A) prior to analysis, ADA at varying concentrations and Alexa labelled DM were premixed and analysed at different time points (0 hours and 2 hours incubation) in the semi-sequential format. For two of the test series unlabelled DM was added at a concentration of 5 µg/ml, while in the other two test series, no unlabelled DM was added.

### Detailed Description of the Invention

### Assay formats for detection of anti-drug antibodies (ADAs).

Herein the term "different formats" refers to affinity assays that differ from each other with respect to at least one, two, three or more features such as: a) the relative order in which the reactants analyte, capturer and detectable reactant (if used) are allowed to react; b) the counterpart relationship between analyte, capturer, and detectable reactant, e.g. if i) the capturer is or is not an affinity counterpart to the detectable reactant and/or to the analyte; ii) the detectable reactant is or is not an affinity counterpart to the analyte and/or to the capturer; iii) the detectable reactant and the capturer are utilizing the same binding site or two spatially spaced binding sites that may be different or equal on the analyte (= different or equal specificities of the detectable reactant and the capturer for binding the analyte, equal means that the analyte is at least bivalent with respect to the binding site concerned); iv) type of counterpart relationship, for instance if one or more of the analyte, capturer and detectable reactant (if used) is or is not an antibody or an antigen/hapten relative to one or more of the other ones of these reactants; c) general-type of affinity assays, i.e. immunoassay, hybridisation assay etc; d) principle utilized for measuring the product, e.g. detectability due to a separately introduced label or an inherently detectable group, due to an affinity group or a signal- generating group etc; e) the state of the solid phase during the capturing of the analyte or a detectable reactant by a solid phase, i.e. porous bed of particles, monolithic porous bed, suspended particles, inner wall of the microcavity etc; f) flow conditions or static conditions during capturing of a reactant such as the analyte or the detectable reactant or when attaching a soluble affinity complex or product to the solid phase; g) Number of reaction steps, for instance one-step or two-step format with respect to the use of the analyte, capturer and detectable reactant; h) limiting or non-limiting amounts/concentrations of the analyte or a counterpart to the analyte.

In a typical affinity assay an uncharacterized amount of an analyte of a sample is allowed to form an affinity complex comprising at least the analyte and an affinity counterpart to the analyte. Depending on the format used, additional affinity reactants may be used and possibly also incorporated into the complex. The amount and type of reactants are selected so that the affinity reactions involved will result in an amount of an affinity complex that will reflect the amount of an analyte in an original or native sample or in the particular sample contacted with one or more of the reactants used.

An assay format used utilizes an immobilized or immobilizable form of the capturer and also a detectable reactant. These two reactants may or may not be the same. Either one or both of the capturer or the detectable reactant will be fully or partly incorporated into an affinity complex the amount of which will reflect the amount of analyte in the sample. The analyte may or may not be part of the complex. Immobilization to a solid phase is used in order to facilitate separate measurement of the portion of the detectable reactant that is incorporated into an affinity complex that reflects the amount of analyte in a sample without disturbing influence from the portion of the detectable reactant that is not incorporated.

An affinity assay/format can be classified with respect to the number of incubations (steps) that is required to form the affinity complex to be measured. A single incubation/step in this context means separately reacting an affinity reactant with a previously formed complex or a single reactant (affinity counterpart to the added reactant).

One-step formats are called "simultaneous" (or "homogenous"), i.e. the analyte, the capturer and the analyte counterpart are reacted in the same incubation/step. Two-step formats, or formats having more than two incubation steps, are called "sequential" formats. Two-step formats (or multi step formats having more than two steps) in which the first step comprises reaction with the immobilized or immobilizable capturer are "forward". If the capturer is not involved in the first step the formats are "reversed".

While inhibition formats are used in other analytical applications, these formats were in general found not to work for the detection of ADAs.

Non-inhibition formats (= non-competitive formats) typically utilize non-limiting amounts of one or more affinity counterparts to the analyte, typically anti-Ans. For certain variants limiting amounts of the counterparts amounts may be used. Amount in this context includes concentration.

The most important non-competitive formats of assays are of the sandwich-type and comprise formation of immobilized or immobilizable complexes in which an analyte is sandwiched between two analyte counterparts, e.g. two anti-Ans, that are directed towards binding sites that are remotely spaced on the same analyte molecule (i.e. allow that both anti-analytes can be bound simultaneously to the analyte). In sandwich assays, one of the analyte counterparts is the detectable reactant and the other one is the capturer. The binding sites on the analyte can be different which implies that the two counterparts used have different specificities. The binding sites involved may in some variants be equal (repetitive, at least bivalent with respect to these binding sites) in the sense that the two counterparts used may react interchangeably with any one of the sites, which also implies that the two counterparts have essentially the same binding specificity. In one embodiment of the invention, the binding sites involved are equal (repetitive, at least bivalent with respect to these binding sites) in the sense that the two counterparts used may react interchangeably with any one of the sites on the analyte, which also implies that the two counterparts to the analyte have, at least essentially, the same binding specificity.

There are two main two-step variants of assay formats, namely: a) the forward format in which the first step comprises incubation of an immobilized or immobilizable analyte counterpart (capturer) with the analyte followed by a second step that comprises incubation of the complex formed in the first step with a second analyte counterpart (the detectable reactant), and b) the reverse format in which the first step comprises incubation of a soluble detectable analyte counterpart (the detectable reactant) with the analyte followed by a second step that comprises incubation of the complex formed in the first step with an immobilized or immobilizable analyte counterpart (capturer).

A simultaneous one-step format of a sandwich assay comprises incubating an immobilized or immobilizable analyte counterpart (capturer), a soluble detectable analyte counterpart (the detectable reactant) and the analyte for the formation of an at least ternary complex that comprises all three of the reactants without separate preformation of a binary complex that comprises only two of the reactants.

In a sandwich format the amount of analyte is preferably determined from the amount of complex (the product) on the solid phase preferably by measuring the detectable reactant on the solid phase after the second step in the two-step variant. In principle measurement of the detectable analyte counterpart (detectable reactant) remaining in the liquid after formation of the product may be feasible.

An analyte in a sandwich assay is at least bivalent (= polyvalent) and has a relatively high molecular weight in order to permit simultaneous binding of two anti-Analytes. The molecular weight thus may be more than, or equal to 1 000 daltons, such as more than, or equal to 10 000 daltons or more than, or equal to 50 000 daltons. These kinds of analytes typically comprise a polymeric structure, such as a biopolymeric structure.

The number of subunits such as different and/or equal amino acid residues, nucleotides, monosaccharide units etc typically is more than, or equal to 10, such as more than, or equal to 100 or more than, or equal to 500.

A sandwich format that utilizes two analyte counterparts (as the capturer and the detectable reactant) that have essentially the same binding specificity are particularly adapted for the assay of analytes that are least bivalent with respect to the binding site utilized on the analyte. One important type of analytes that complies with this condition is an at least bivalent antibody and the assay formats concerned may thus be used for the assay of antigen-specific antibodies irrespective of class, subclass or species. In this kind of sandwich format, limiting amounts and/or selected densities of the capturer are typically advantageous in order to secure simultaneous binding of the two counterparts to the same analyte molecule. This in particular applies to the counterpart bound to the solid phase (capturer). Careful selection of the amount of the detectable counterpart is also appropriate in order to avoid disturbing formation of soluble ternary complexes comprising the analyte sandwiched between two detectable analyte counterparts. For more details see PCT/SE2006/000071 (Gyros Patent AB).

Sandwich formats that utilize two analyte counterparts, such as anti-An1 and anti-An2, (as the capturer and the detectable reactant), that have different binding specificities are in particular adapted for analytes that expose two different binding sites that are spatially spaced. This kind of formats can in principle be used for measuring any larger biomolecule that is polyvalent and comprises one or more of the structures given below for reactants in general. The format is particularly well-adapted for measuring a particular subpopulation of a group of substances where each subpopulation has a) a binding site (common binding site) that also is present in the other subpopulations, and b) another different binding site that does not exist in any of the other subpopulations. The sandwich format referred to in this paragraph (IAA) can be illustrated with the assay of A) an antigen-specific antibody of a certain Ig-class, Ig-subclass, species etc (one counterpart is an antigen/hapten and the other is an anti-Ig-class antibody, anti-Ig-subclass antibody, or some other Ig-binding reactant), B) An analyte being an antigen-specific antibody analyzed by using saturating amounts of a capture reagent that is specific for an immunoglobulin class (IgG, IgA, IgM, IgD or IgE) or immunoglobulin subclass (e.g. IgG1, IgG2, IgG3, IgG4, IgA1, IgA2) or some other Ig binding reagent reacting with the corresponding immunoglobulin class or subclass followed by addition of a detectable antigen preparation that binds to specific antibodies belonging to the capture class or subclass of immunoglobulins.

Another non-competitive format utilizes only one analyte counterpart, which is in immobilized or immobilizable form. In this case complex formation leads to an immobilized complex, or a soluble complex that subsequently is immobilized. The immobilized complex as such is then measured. This kind of non-inhibition formats can be of the one-step type. The immobilized or immobilizable counterpart may be detectable, e.g. comprise a signal-generating label for which the signal is changed as a consequence of binding to the analyte (coinciding capturer and detectable reactant).

In preferred embodiments of the invention, two-step formats of the reversed type are used. In the reversed format, the first step comprises incubation of a soluble detectable analyte counterpart (the detectable reactant) with the analyte followed by a second step that comprises incubation of the complex formed in the first step with an immobilized or immobilizable analyte counterpart (capturer).

An individual reactant used is typically selected among members of ligand-receptor pairs being affinity counterparts, such as a) antigens/haptens, b) antibodies or antigen/hapten-binding fragments thereof including affinity reactants mimicking the antigen/hapten-binding ability of antibodies and their antigen/hapten-binding fragments, c) hormones, such as of steroid structure or peptide structure and hormone receptors.

An affinity reactant used in the context of the invention typically exhibits one or more structures selected among members of the group consisting of: a) amino acid structures including protein structures such as peptide structures such as poly and oligopeptide structures, such as antibodies, and including mimetics and chemically modified forms of these structures and/or other structures of organic or bio-organic nature including drugs.

Bridging immunoassay (BIA) involves at least three interacting molecules: A capture reagent (1), an analyte (2), and a detection reagent (3). In bridging immunoassay (BIA), the analyte molecule (2) binds to both the capture reagent (1), and to the detection reagent (2), to form a complex of the type capture reagent (1) - analyte (2) - detection reagent (3), thus, the analyte (2) forms a bridge between the capture reagent (1) and the detection reagent (3).

The indirect antibody assay (IAA) and reversed antibody assay (RAA) can also sometimes be used depending on reagent properties, sample origin and properties of the DM. In this case the preferred assay format in Gyrolab is the sequential procedures utilizing excess amounts of reagents for capturing and detection.

When samples are to be analysed containing anti-drug antibodies (ADAs) directed against bivalent therapeutic antibodies, the previously preferred assay format (Fig. 1) has been to premix equimolar concentrations of capture and detecting reagents with the sample, to enable formation of complexes composed of the three interactants (i.e. a capture molecule (which can be a drug molecule, DM), an anti-drug antibody (ADA), and a detecting molecule (which can be a labelled drug molecule, DM-A)) that can be detected. If the capture molecule is biotinylated (B-DM), the complex can be captured on immobilised streptavidin. There are several drawbacks with this procedure:
- Theoretically only 50 % of the formed complexes can be detected (the outcome from mixing the reagents follows the binomial theorem)
- At high concentrations of anti-drug antibodies (ADAs), the assay will show typical signs of prozone effects which will translate into pronounced hook effects in the assay
- The amounts of samples and reagents required for efficient mixing is relatively large
- The time required for forming stable complexes exceed several hours and may, for practical reasons, proceed over night
- The exposure of naked streptavidin for Alexa labelled detecting reagent will contribute to increase background in the assay

In the real world, samples that are analysed for presence of ADA often also contain significant amounts of DM. Under these circumstances it is likely that immune complexes composed of ADA and DM are formed. This will complicate the detection of ADA. Sometimes preformed immune complexes are dissociated by acid treatment followed by necessary steps to mix in labelled components into samples followed by adjustment to neutral pH to incorporate labelled components in the immune complex (Lofgren JA, Dhandapani S, Pennucci JJ, Abbott CM, Mytych DT, Kaliyaperumal A, Swanson SJ, Mullenix MC. Comparing ELISA and surface plasmon resonance for assessing clinical immunogenicity of panitumumab. J. Immunol. 2007, 178, 7467-7472). Alternatively components required for the assay are mixed with samples only and incubated for very long time prior to analysis, to allow exchange reactions and incorporation of reactants necessary for detection of ADA.

Furthermore, ADA may differ in affinity for DM. Since it is considered important to detect all signs of immunogenicity, also low affinity antibodies should be detected. Paradoxically, low affinity ADA might be more accessible in assay situations where the sample also contains DM since low affinity ADA are more likely to be present in non-complexed form than high affinity ADA. This may simplify the formation of detectable immune complexes. The stability of complexes is dependent upon the dissociation properties of the ADA but dissociation depends essentially only on time (s⁻¹). Thus assays requiring short process time to reach completion are more likely to also detect low affinity ADA since these have had less time to dissociate prior to detection. Thus assays requiring short assay times are likely to better detect low affinity ADA.

As indicated above the currently preferred procedure to measure ADA is to premix all three reaction components. The main reason is that ADA having specificity for the paratope of the DM is expected to react as an anti-idiotypic antibody with a presumed preference to form complexes with DM at a 1:1 ratio where Fab arms of ADA interact with both Fab arm of DM. If such complexes are formed it will be difficult to introduce labelled (biotin- or alexa-labelled) DM to generate detectable signal from the complex. Consequently methods devised for assaying ADA usually depend on premixing of reagents for sufficient time to form stable, detectable complexes by eventually capturing the complex through the biotinylated compound in the complex to streptavidin beads.

We have used a model system based on a mouse monoclonal antibody (DM analogue) directed against human Carcino embryonic antigen (CEA) which can form a complex together with an anti-idiotypic antibody (ADA analogue) to simulate the most exclusive specificity of an ADA response against a DM analogue.

This model system has been modified in terms of proportion of reagents and in various environments in a completely homogenous format to generate a "standard curve" displaying the relationship between ADA concentration and assay response. Furthermore, we have taken the selected reagent concentrations after mixing and used also for other assay protocols. All reactions have been performed in Exxip F (Gyros AB, Uppsala, Sweden) comprising a detergent (0.1 % Tween 20, *i.e.* polyoxyethylenesorbitan monolaurate, Sigma, USA).

In the following, three different assay procedure formats for detection of ADAs are discussed. The first procedure, outlined in Fig. 1, is in the following called the homogenous procedure. The second procedure, outlined in Fig. 2, is in the following called the sequential procedure. The third procedure, outlined in Fig. 3, is in the following called the semi-sequential procedure.

### Homogenous procedure

Fig. 1 outlines a homogenous assay procedure wherein a) capture reagent (1), analyte (2), and detection reagent (3) is premixed, the capture reagent (1) and the detection reagent (3) have equimolar concentrations, while the concentration of the analyte (2) can be varied; and b) the capture reagent (1), analyte (2), and detection reagent (3) forms a complex; and c) the complex is captured on a substrate (4). The capture reagent (1) can be a biotinylated drug molecule (1) comprising a capturing moiety (1b) and a drug molecule (1a). The analyte (2) can be an anti-drug antibody (ADA). The detection reagent (3) can be an Alexa-labelled drug molecule (3) comprising a detectable Alexa label (3b) and a drug molecule (3a). The following homogenous procedure was tested experimentally:
Biotinylated DM (0.25 µg/ml), ADA (varying concentration levels tested), and Alexa-labelled DM (0.25 µg/ml) were premixed to form a complex, and the complex was added to a substrate (4), which in this case was a streptavidin column. The complex binds to the streptavidin surface via the biotin label and can be detected, as the complex also comprises a detectable Alexa-label.

### Sequential procedure.

Fig. 2 outlines a sequential assay procedure wherein a) a substrate (4) is used to b) bind a capture reagent (1) added thereto, in the next step c) an analyte (2) is added, and the analyte (2) binds to the capture reagent (1) immobilized on the substrate (4), in a further step d) a detection reagent (3) is added, and the detection reagent (3) binds to the analyte (2) that has previously been bound to the capture reagent (1) immobilized on the substrate (4). The capture reagent (1) can be a biotinylated drug molecule (1) comprising a capturing moiety (1b) and a drug molecule (1a). The analyte (2) can be an anti-drug antibody (ADA). The detection reagent (3) can be an Alexa-labelled drug molecule (3) comprising a detectable Alexa label (3b) and a drug molecule (3a). The substrate (4) can be a streptavidin surface in a column. The following sequential procedure was tested experimentally:
A streptavidin column was saturated by applying saturating concentrations (100 µg/ml) of biotinylated drug molecule on the column, so that the biotinylated drug molecule binds to the streptavidin column via the biotin label (Fig. 2 a-b). In the next step varying concentration levels of an anti-drug antibody (an anti-drug antibody is defined as an antibody to the drug molecule) were applied to the streptavidin column, so that the anti-drug antibody binds to the biotinylated drug molecule immobilized on the streptavidin column (Fig. 2c). In the next step Alexa labelled drug molecules (0.25 µg/ml) were added to the streptavidin column, so that the Alexa labelled drug molecules binds to the anti-drug antibody immobilized on the streptavidin column via the biotinylated drug molecule (Fig. 2d).

### Semi-sequential procedure.

Fig. 3 outlines a semi-sequential assay procedure wherein a) a capture reagent (1) is added to a substrate (4); and b) the capture reagent (1) binds to the substrate (4) and is thereby immobilized; and c) an analyte (2) is mixed with a detection reagent (3); and d) the analyte (2) binds to the detection reagent (3) to form a complex (5); and e) the complex (5) is added to the capture reagent (1) immobilized on the substrate (4), so that the complex (5) binds to the capture reagent (1) immobilized on the substrate (4). The capture reagent (1) can be a biotinylated drug molecule (1) comprising a capturing moiety (1b) and a drug molecule (1a). The analyte (2) can be an anti-drug antibody (ADA). The detection reagent (3) can be an Alexa-labelled drug molecule (3) comprising a detectable Alexa label (3b) and a drug molecule (3a). The substrate (4) can be a streptavidin surface in a column. The following semi-sequential procedure was tested experimentally:
A streptavidin column was saturated by applying saturating concentrations (100 µg/ml) of biotinylated drug molecule on the column, so that the biotinylated drug molecule binds to the streptavidin column via the biotin label (Fig. 3 a-b). In the next step varying concentration levels of an anti-drug antibody (an anti-drug antibody is defined as an antibody to the drug molecule) were premixed (Fig. 3c) with constant amounts (0.25 µg/ml) of Alexa labelled drug molecule (3) to form a complex (Fig. 3d, item 5) comprising the anti-drug antibody (2) and the Alexa labelled drug molecule (3). In the next step the complex (5) was added to the streptavidin column, so that the complex (5) binds to the streptavidin column via the biotinylated drug molecule (1) immobilized on the streptavidin column.

The results of the 3 experiments comparing results from the use of the homogenous procedure, the sequential procedure, and the semi-sequential procedure are displayed in Fig. 4. Fig. 4 outlines dose-response curves for the different assay protocols (the homogenous procedure, the sequential procedure, and the semi-sequential procedure) using the same set up ofimmunoreagents. The concentration levels of the reagents used were the same for all three procedures.

As illustrated in Fig. 4, the slopes of the curves differ significantly over the working range of the assay. The semi-sequential format provides several advantages versus the other alternatives:
- The curve of the sequential format is shifted towards lower concentrations of anti-drug antibody (ADA) thus providing a more sensitive assay compared to the homogeneous format (2-3 times improvement of sensitivity in the low end of the curve)
- The background (noise level) of the semi-sequential format is reduced compared to the homogeneous format, presumably because of less interactions between Alexa labelled drug molecule and naked Streptavidin
- Based on these experiments it seems that the hook effect is less pronounced in the semi-sequential format compared to the homogeneous format

An important aspect of analyzing samples for presence of ADA is the assay tolerance for presence of drug molecule (DM) in sample. In order to evaluate the semi-sequential assay procedure for presence of ADA, samples were prepared to contain varying amounts of ADA and DM at 1 and 5 µg/ml, respectively and incubated for 2 hours to form complexes. Subsequently Alexa labelled DM was added (0.25 µg/ml) followed by analysis in the semi-sequential format. The results (Fig. 5a) indicate that the assay drug tolerance is only marginally affected in this assay system compared to the same experimental conditions analysed in the homogeneous format (Fig. 5b).

Fig. 5a) and 5b) outlines the drug tolerance (i.e. the impact of drug molecules in the sample upon the test result for detection of ADA) in semi-sequential (Fig. 5a)) and homogeneous (Fig. 5b)) formats.

Yet another aspect of assaying samples for ADA is the time required to perform pre-incubations between labelled reagents and samples containing ADA to form sufficiently stable complexes to endure analysis and still obtain assays with high sensitivity for ADA. In order to evaluate the time requirements for pre-incubating, ADA at varying concentrations and Alexa labelled DM was premixed and analysed at different time points in the semi-sequential format. Fig. 6 outlines the effect of time for pre-incubation of ADA and Alexa-labelled DM in the semi-sequential procedure format. For practical reasons an additional 10-20 min incubation time has to be added to the 0- and 2 hrs incubation indicated. As seen from Fig. 6, it appears that the use of low incubation times is favourable as this provides higher signal to noise ratios for the semi-sequential procedure format.

One possible effect of increasing the time for pre-incubation is illustrated in Fig. 7. In Fig. 7a)-c) a complex (5) has been formed between the analyte (2) and the detection reagent (3), if there is an excess of the detection reagent (3) compared to analyte (2), and/or if the mixing time for mixing the analyte (2) and the detection reagent (3) is relatively long, the complex (5) may bind to another unit of the detection reagent (3) to form a complex comprising two units of the detection reagent (3) and one unit of the analyte (2) (Fig. 7b)), and, as shown in c), such a complex will not bind to a capture reagent (1) immobilized on a substrate (4). The capture reagent (1) can be a biotinylated drug molecule (1) comprising a capturing moiety (1b) and a drug molecule (1a). The analyte (2) can be an anti-drug antibody (ADA). The detection reagent (3) can be an Alexa-labelled drug molecule (3) comprising a detectable Alexa label (3b) and a drug molecule (3a). The substrate (4) can be a streptavidin surface in a column.

A further possible effect of increasing the time for pre-incubation is illustrated in Fig. 8. In Fig. 8a) a bivalent analyte (2) binds to a detection reagent (3) via one of the two binding sites available on the analyte (2). In Fig. 8b) the bivalent analyte (2) bound to the detection reagent (3) in Fig. 8a) is given time to proceed with the binding process, so that it binds with both of the two binding sites to the detection reagent (3). In Fig. 8c) the bivalent analyte (2) bound to the detection reagent (3) via both of the two binding sites will be unable to bind to a capture reagent (1) immobilized on a substrate (4). The capture reagent (1) can be a biotinylated drug molecule (1) comprising a capturing moiety (1b) and a drug molecule (1a). The analyte (2) can be an anti-drug antibody (ADA). The detection reagent (3) can be an Alexa-labelled drug molecule (3) comprising a detectable Alexa label (3b) and a drug molecule (3a). The substrate (4) can be a streptavidin surface in a column.

Fig. 9 outlines the sensitivity for the hook effect in the upper end of the concentration range for the homogeneous and semi-sequential formats, respectively. As seen from Fig. 9, the hook effect is more pronounced and appears at lower ADA concentration levels for the homogenous procedure compared to the semi-sequential procedure.

Fig. 10 outlines the dependence of capture column capacity for assay performance in the semi-sequential format. The figures in the panel represents the proportions of B-DM vs B-BSA (100, 50/50, 25/75 and 10/90 %, respectively) on the capture column. As seen from Fig. 10, it appears that a high column capacity provides higher signal to noise ratios for the semi-sequential procedure format.

Fig. 11a) illustrates a substrate (4) binding two units of a capture reagent (1), the substrate (4) is not saturated with capture reagent (1), therefore a part of the surface of substrate (4) is not covered with the capture reagent (1). As illustrated by the arrow, a complex formed between the analyte (2) and the detection reagent (3) can interact with the substrate (4) and even bind non-specifically to the substrate (4) and thereby increase the background signal in the system. Accordingly, excess amounts of the detection reagent (3) can interact with the substrate (4) and even bind non-specifically to the substrate (4) and thereby increase the background signal in the system. In applications wherein the substrate (4) is situated in a column the inventors have observed that the capture reagent (1) may, under certain conditions, tend to bind only to a portion of the substrate (4) situated close to the inlet portion of the column, so that the portion of the substrate (4) situated further downstream in the column is not covered with the capture reagent (1). On the contrary, the portion of the substrate (4) situated further downstream in the column remains exposed to bind, for example non-specifically, to any of the other reagents used in the assay procedure. This is a disadvantage, as this may lead to increased background signals, for example from excess amounts of detection reagent (3) adsorbed to the substrate (4).

In Fig. 11b) a substrate (4) has been contacted with a saturating solution of the capture reagent (1), so that the distance between the capture reagent (1) units immobilized on the substrate (4) is minimized. In other words, the surface of the substrate (4) has been saturated with the capture reagent (1). Thereby the surface of the substrate (4) available for non-specific interaction with, for example, the complex formed between the analyte (2) and the detection reagent (3), is minimized.

Fig.12 shows the results from an evaluation of drug tolerance and the influence of incubation time in a semi-sequential assay procedure. In order to evaluate the time requirements for pre-incubation of ADA and Alexa labelled DM (DM-A) prior to analysis, ADA at varying concentrations and Alexa labelled DM were premixed and analysed at different time points (0 hours and 2 hours incubation) in the semi-sequential format. For two of the test series unlabelled DM was added at a concentration of 5 µg/ml, while in the other two test series, no unlabelled DM was added. As seen from Fig. 12, it appears to be an advantage to minimize the time for pre-mixing, as this provides the highest signal to noise ratios both for the test series without added DM, and for the test series wherein DM was added.

In one embodiment of the invention the substrate (4) comprises particles that are porous, or semi-pellicular, or pellicular *(i.e.* non porous).

In a further embodiment of the invention the particles are essentially circular and have an average diameter higher than 10 µm. In one embodiment of the invention the particles have an average diameter within the range 10-14 µm. In a further embodiment of the invention the particles have an average diameter of 15 ± 0.5 µm.

In a further embodiment of the invention the particles have a homogenous, or narrow, size distribution.

Yet another aspect of running the assay in the semi-sequential manner is that the distribution of bound complexes will not depend on the interaction between streptavidin and biotin of the DM in the complex but rather the interaction between a capturing DM and an ADA that is part of a detectable complex. This difference has a potential to resolve variable affinities of different samples containing ADA but require more studies for implementation, particularly using model systems with different affinities between the interacting components.

In one embodiment of the invention, the method is a semi-sequential assay procedure wherein a) a capture reagent (1) is added to a substrate (4); and b) the capture reagent (1) binds to the substrate (4) and is thereby immobilized; and c) an analyte (2) is mixed with a detection reagent (3); and d) the analyte (2) binds to the detection reagent (3) to form a complex (5); and e) the complex (5) is added to the capture reagent (1) immobilized on the substrate (4), so that the complex (5) binds to the capture reagent (1) immobilized on the substrate (4).

In one embodiment of the invention, the capture reagent (1) can be a biotinylated drug molecule (1) comprising a capturing moiety (1b) and a drug molecule (1a). The analyte (2) can be an anti-drug antibody (ADA). The detection reagent (3) can be an Alexa-labelled drug molecule (3) comprising a detectable Alexa label (3b) and a drug molecule (3a). The substrate (4) can be a streptavidin surface in a column.

The anti-drug antibody (ADA) can be a part of a sample of biological origin, for example blood, serum, plasma, urine, or tissue from humans or animals. By definition an anti-drug antibody (ADA) is an antibody that is an affinity counterpart to a drug molecule. The anti-drug antibody (ADA) can be an anti-idiotypic antibody to the drug molecule, but in other variants the ADA can have affinity towards other structural parts of the drug molecule than the Fab-arms. The anti-drug antibodies are polyclonal in nature and can vary in affinity towards the drug molecule, they can belong to different immunoglobulin subclasses, and they can be present in samples in a range of different concentrations.

In a further embodiment of the invention, the analyte (2) can be a drug molecule (for example an antibody), the capture reagent (1) can be a biotinylated anti-idiotypic antibody to the drug molecule, and the detection reagent (3) can be an Alexa-labelled anti-idiotypic antibody to the drug molecule. In this embodiment, the capture reagent (1) comprises biotin as a capturing moiety (1b) and an anti-idiotypic antibody (1a) to the drug molecule. Further, the detection reagent (3) comprises a detectable Alexa label (3b), and an anti-idiotypic antibody (3a) to the drug molecule. The substrate (4) can be a streptavidin surface in a column.

The results indicate that shorter premixing times paradoxically increases the sensitivity for detection of ADA, hypothetically because in the complex that is formed, fewer and fewer of accessible ADA sites are available by increasing time. One may say that the complex matures over time. This indicates that it may be preferable to control the time used for each procedure step accurately, and that it may be preferable to minimize the incubation time used in each step.

Thus CDs containing functions for mixing upstream of the capture column, appropriate volumes of sample containing ADA and Alexa labelled DM can be volume defined, mixed by an appropriate "releasing" spin for a sufficiently long period of time before simultaneously "releasing" the volume defined samples to the capture column. This increases the convenience in several ways:
- The time required for pre-incubation and analysis could be significantly reduced
- The sample volumes required to perform premixing in CD can be reduced compared to performing premixing in MTP wells
- Last by not least, the process of pipetting samples and reagents can be handled completely automatically, relieving repeated operator interventions during processing.

WO 2005/094976 A1 (Gyros Patent AB) discloses a mixing unit which is applicable as a part of a device in one embodiment of the present invention.

### Material and Methods

This model system is based on mouse monoclonal antibodies from Erfa Biotech, Canada. The DM analogue is an anti Carcino embryonic antigen (cat # 76050) and the ADA analogue is an anti-idiotypic antibody to anti Carcino embryonic antigen (cat# 76150). The DM is Biotin labelled to work as a capture antibody and Alexa 647 labelled as a detection antibody. All analyses were run with a Gyrolab Workstation (Gyros AB) in a Bioaffy200 CD micro laboratory (Gyros AB).

### Preparation of capture- and detection antibody

The DM was labelled with EZ-Link Sulfo-NHS-LC-Biotin (cat # 21335, PIERCE Biotechnology) and Alexa Flour 647 (cat # A-20186, Molecular Probes) respectively according to the suppliers instructions.

### Homogenous assay procedure

With a homogenous assay procedure all three components, Biotin labelled DM, Alexa labelled DM and ADA, are premixed prior to analysis. As the same volumes of all three components are premixed, the concentrations at mixing have to be three times the desired final concentrations. All dilutions were performed in Exxip F (Gyros AB).

The Biotin labelled DM and the Alexa labelled DM were diluted to 0.75 µg/ml respectively. The ADA analogue was diluted to varying concentrations between 0.21 - 3600 ng/ml to generate a "standard curve". Ten micro litres of each component were premixed in a micro plate and incubated with constant shaking for 2 hours at room temperature.

### Sequential assay procedure

With a sequential assay procedure the three components are added in series starting with the Biotin labelled DM followed by addition of ADA and finally addition of the Alexa labelled DM.

The biotin labelled DM was diluted to 100 µg/ml in PBS-T (15 mM phosphate buffer, 150 nM NaCl, 0.02 % NaN₃, 0.02 % Tween 20) and the Alexa labelled DM was diluted to 0.25 µg/ml in Exxip F. The ADA analogue was diluted in Exxip F to varying concentrations between 0.29 - 300 ng/ml to generate a "standard curve".

### Semi-sequential assay procedure

With a semi-sequential assay the Biotin labelled DM is added at a saturating concentration followed by the addition of premixed Alexa labelled DM and ADA. The premixing is done with equal volumes and for this reason the concentrations at mixing have to be two times the desired final concentrations.

The Alexa labelled DM was diluted to 0.5 µg/ml in Exxip F and the ADA was diluted to varying concentrations between 0.14 - 2400 ng/ml to generate a "standard curve".
Ten micro litres of each component was mixed in a micro plate and incubated with constant shaking for 2 hours at room temperature. The assay was also run without incubation, with the Alexa labelled DM and ADA just mixed right before analysis. The Biotin labelled DM was diluted to 100 µg/ml in PBS-T in connection to the assay start*.

*In the column capacity assay for the semi-sequential format, 100 µg/ml of Biotin labelled DM was mixed with varying proportions of 100 µg/ml of Biotin labelled BSA.

### Drug tolerance

To generate a "sample" the ADA analogue was mixed with equal volumes of unlabelled DM and incubated for 2 hours shaking at room temperature. The ADA was diluted in Exxip F to varying concentrations to generate a "standard curve" and the DM was diluted to a final concentration of 0, 1 and 5 µg/ml. Drug tolerance was run both in a homogenous and in a semi-sequential assay format.

In the homogenous format the "sample" was mixed in a micro plate with Biotin labelled DM and Alexa labelled DM. Incubation took place for 2 hours shaking at room temperature. The final concentration of each labelled DM was 0.25 µg/ml.

In the semi-sequential format, the "sample" was mixed with the Alexa-labelled DM and incubated for 2 hours shaking at room temperature. The final concentration of Alexa labelled DM was 0.25 µg/ml and the Biotin labelled DM was diluted to 100 µg/ml right before analysis.

## Claims

1. Method for detection of an analyte (2) in a sample, wherein the method comprises the following sequence of steps:
a) a solution of a capture reagent (1), wherein the capture reagent (1) comprises a capturing moiety (1b) and an affinity counterpart (1a) to the analyte (2), is contacted with a substrate (4) having a surface with reactive sites capable of binding to the capturing moiety (1b); whereby the capture reagent (1) binds to the substrate (4) via the capturing moiety (1b) and is immobilized, until said reactive sites are saturated with the immobilized capture reagent (1);
b) the sample comprising the analyte (2) is contacted with a detection reagent (3), wherein the detection reagent (3) comprises a detectable moiety (3b) and an affinity counterpart (3a) to the analyte (2); whereby the analyte (2) binds to the detection reagent (3) via the affinity counterpart (3a) to form a complex (5);
c) the substrate (4) is exposed to the complex (5), whereby the complex (5) binds via the analyte (2) to the capture reagent (1) immobilized on the substrate (4); and
d) the detectable moiety (3b) is detected,
wherein the analyte (2) is an anti-drug antibody, the capture reagent (1) comprises a drug molecule (1a) linked to the capturing moiety (1b), and the detection reagent (3) comprises a drug molecule (3a) linked to the detectable moiety (3b),
**characterized in that** the substrate (4) is provided in a column, and that the drug molecule (3a) is an antibody.

2. Method according to claim 1, further comprising a step of washing the substrate (4) with a washing solution prior to step d).

3. Method according to claim 1 or 2, wherein the washing solution comprises a detergent at a concentration level less than 5%, preferably less than 1%, more preferably less than 0.5%, wherein the detergent preferably is non-ionic, especially polyoxyethylenesorbitan monolaurate.

4. Method according to any one of the preceding claims, wherein the sample is contacted with the detection reagent (3) for a time less than 24 hours prior to step c), preferably less than 2 hours prior to step c), more preferably less than 20 minutes prior to step c), more preferably less than 10 minutes prior to step c), more preferably less than 1 minute prior to step c).

5. Method according to any one of the preceding claims, wherein the capturing moiety (1b) is biotin and the substrate (4) having the surface with reactive sites comprises streptavidin.

6. Method according to any one of the preceding claims, wherein the detectable moiety (3b) is selected from a group of reagents consisting of: Alexa Fluor 633, Alexa Fluor 647, DyLight 649, Cy-5, Cy-5.5, and fluorescent nanoparticles.

7. Method according to any one of the preceding claims, wherein the sample has a volume less than, or equal to, 1000 nl, preferably less than, or equal to, 200 nl.

8. Method according to any one of the preceding claims, wherein the detection reagent (3) has a volume less than, or equal to, 1000 nl, preferably less than, or equal to, 200 nl.

9. Method according to any one of the preceding claims, wherein the detection reagent (3) has a concentration less than 15 µg/ml, or less than 100 nM, preferably less than 0.5 µg/ml, or less than 3.3 nM.

10. Method according to any one of the preceding claims, wherein the substrate (4) comprises particles that are porous, or semi-pellicular, or pellicular (i.e. non porous), wherein the particles preferably are essentially circular and have an average diameter higher than 10 µm, and/or wherein the particles preferably have a homogenous size distribution.

11. Method according to any one of the preceding claims, wherein the substrate (4) is a part of a device, wherein the device comprises:
- a sample inlet; and
- a detection reagent inlet; and
- a capture reagent inlet; and
- a mixing chamber in fluid contact with the sample inlet and the detection reagent inlet, wherein the mixing chamber is positioned in a direction downstream of the sample inlet and the detection reagent inlet; and
- a column comprising the substrate (4), wherein the column is in fluid contact with the sample inlet, the detection reagent inlet, the capture reagent inlet, and the mixing chamber, and wherein the column is positioned in a direction downstream of the capture reagent inlet and the mixing chamber; and
- an outlet in fluid contact with the column, wherein the outlet is positioned in a direction downstream of the column; and
- a means for detecting the detection reagent.

## Patentansprüche

1. Verfahren zum Nachweisen eines Analyten (2) in einer Probe, wobei das Verfahren die folgende Abfolge von Schritten umfasst:
a) eine Lösung eines Capture-Reagenzes (1), wobei das Capture-Reagenz (1) eine Capturing-Einheit (1 b) und einen Affinitäts-Gegenspieler (1a) zu dem Analyten (2) umfasst, wird mit einem Substrat (4) in Kontakt gebracht, welches eine Oberfläche mit reaktionsfähigen Stellen aufweist, die fähig sind, an die Capturing-Einheit (1 b) zu binden; wodurch das Capture-Reagenz (1) mittels der Capturing-Einheit (1 b) an das Substrat (4) bindet und immobilisiert wird, bis die reaktionsfähigen Stellen mit dem immobilisierten Capture-Reagenz (1) gesättigt sind;
b) die Probe, umfassend den Analyten (2), wird mit einem Nachweisreagenz (3) in Kontakt gebracht, wobei das Nachweisreagenz (3) eine nachweisbare Einheit (3b) und einen Affinitäts-Gegenspieler (3a) zu dem Analyten (2) umfasst; wodurch der Analyt (2) mittels des Affinitäts-Gegenspielers (3a) an das Nachweisreagenz (3) bindet, um einen Komplex (5) zu bilden;
c) das Substrat (4) wird dem Komplex (5) ausgesetzt, wodurch der Komplex (5) mittels des Analyten (2) an das auf dem Substrat (4) immobilisierte Capture-Reagenz (1) bindet; und
d) die nachweisbare Einheit (3b) wird nachgewiesen,
wobei es sich bei dem Analyten (2) um einen Anti-Arzneimittel-Antikörper handelt, das Capture-Reagenz (1) ein Arzneimittelmolekül (1a) umfasst, das mit der Capturing-Einheit (1 b) verknüpft ist, und das Nachweisreagenz (3) ein Arzneimittelmolekül (3a) umfasst, das mit der nachweisbaren Einheit (3b) verknüpft ist,
**dadurch gekennzeichnet, dass** das Substrat (4) in einer Säule bereitgestellt wird und dass es sich bei dem Arzneimittelmolekül (3a) um einen Antikörper handelt.

2. Verfahren gemäß Anspruch 1, ferner umfassend einen Schritt, bestehend im Waschen des Substrats (4) vor Schritt (d) mit einer Waschlösung.

3. Verfahren gemäß Anspruch 1 oder 2, wobei die Waschlösung ein Detergenz in einem Konzentrationsspiegel von weniger als 5%, vorzugsweise weniger als 1 %, stärker bevorzugt weniger als 0,5% umfasst, wobei es sich bei dem Detergenz vorzugsweise um ein nicht-ionisches, insbesondere Polyoxyethylensorbitanmonolaureat, handelt.

4. Verfahren gemäß einem der vorhergehenden Ansprüche, wobei die Probe für eine Zeit von weniger als 24 Stunden vor Schritt c) mit dem Nachweisreagenz (3) in Kontakt gebracht wird, vorzugsweise weniger als 2 Stunden vor Schritt c), stärker bevorzugt weniger als 20 Minuten vor Schritt c), stärker bevorzugt weniger als 10 Minuten vor Schritt c), stärker bevorzugt weniger als 1 Minute vor Schritt c).

5. Verfahren gemäß einem der vorhergehenden Ansprüche, wobei es sich bei der Capturing-Einheit (1b) um Biotin handelt und das Substrat (4), welches die Oberfläche mit reaktionsfähigen Stellen aufweist, Streptavidin umfasst.

6. Verfahren gemäß einem der vorhergehenden Ansprüche, wobei die nachweisbare Einheit (3b) aus einer Gruppe von Reagenzien ausgewählt ist, bestehend aus: Alexa Fluor 633, Alexa Fluor 647, DyLight 649, Cy-5, Cy-5.5 und fluoreszierenden Nanopartikeln.

7. Verfahren gemäß einem der vorhergehenden Ansprüche, wobei die Probe ein Volumen von weniger als oder gleich 1000 nl aufweist, vorzugsweise weniger als oder gleich 200 nl.

8. Verfahren gemäß einem der vorhergehenden Ansprüche, wobei das Nachweisreagenz (3) ein Volumen von weniger als oder gleich 1000 nl aufweist, vorzugsweise weniger als oder gleich 200 nl.

9. Verfahren gemäß einem der vorhergehenden Ansprüche, wobei das Nachweisreagenz (3) eine Konzentration von weniger als 15 µg/ml, oder weniger als 100 nM, vorzugsweise weniger als 0,5 µg /ml oder weniger als 3,3 nM aufweist.

10. Verfahren gemäß einem der vorhergehenden Ansprüche, wobei das Substrat (4) Teilchen umfasst, die porös, oder semi-membranartig, oder membranartig (d.h. nicht porös) sind, wobei die Teilchen vorzugsweise im Wesentlichen kreisförmig sind und einen durchschnittlichen Durchmesser von mehr als 10 µm aufweisen und/oder wobei die Teilchen vorzugsweise eine homogene Größenverteilung aufweisen.

11. Verfahren gemäß einem der vorhergehenden Ansprüche, wobei das Substrat (4) Teil einer Vorrichtung ist, wobei die Vorrichtung umfasst:
- einen Probeneingang; und
- einen Eingang für das Nachweisreagenz; und
- einen Eingang für das Capture-Reagenz; und
- eine Mischkammer in Flüssigkeitskontakt mit dem Probeneingang und dem Eingang für das Nachweisreagenz, wobei die Mischkammer in einer Richtung stromabwärts von dem Probeneingang und dem Eingang für das Nachweisreagenz angebracht ist; und
- eine Säule, umfassend das Substrat (4), wobei die Säule in Flüssigkeitskontakt mit dem Probeneingang, dem Eingang für das Nachweisreagenz, dem Eingang für das Capture-Reagenz und der Mischkammer ist und wobei die Säule in einer Richtung stromabwärts von dem Eingang für das Capture-Reagenz und von der Mischkammer angebracht ist; und
- einen Ausgang in Flüssigkeitskontakt mit der Säule, wobei der Ausgang in einer Richtung stromabwärts von der Säule angebracht ist; und
- ein Mittel zum Nachweisen des Nachweisreagenzes.

## Revendications

1. Procédé de détection d'un analyte (2) dans un échantillon, dans lequel le procédé comprend la séquence d'étapes suivante :
a) une solution de réactif de capture (1) dans laquelle le réactif de capture (1) comprend un fragment de capture (1b) et une contrepartie d'affinité (1a) envers l'analyte (2), est mise en contact avec un substrat (4) ayant une surface comportant des sites réactifs capables de se lier au fragment de capture (1b) ; moyennant quoi le réactif de capture (1) se lie au substrat (4) par l'intermédiaire du fragment de capture (1b) et est immobilisé, jusqu'à ce que lesdits sites réactifs soient saturés par le réactif de capture (1) immobilisé ;
b) l'échantillon comprenant l'analyte (2) est mis en contact avec un réactif de détection (3), dans lequel le réactif de détection (3) comprend un fragment détectable (3b) et une contrepartie d'affinité (3a) envers l'analyte (2) ; moyennant quoi l'analyte (2) se lie au réactif de détection (3) par l'intermédiaire de la contrepartie d'affinité (3a) de manière à former un complexe (5) ;
c) le substrat (4) est exposé au complexe (5), moyennant quoi le complexe (5) se lie par l'intermédiaire de l'analyte (2) au réactif de capture (1) immobilisé sur le substrat (4) ; et
d) le fragment détectable (3b) est détecté,
dans lequel l'analyte (2) est un anticorps anti-médicament, le réactif de capture (1) comprend une molécule médicamenteuse (1a) liée au fragment de capture (1b), et le réactif de détection (3) comprend une molécule médicamenteuse (3a) liée au fragment détectable (3b) ;
**caractérisé en ce que** le substrat (4) est utilisé dans une colonne, et **en ce que** la molécule médicamenteuse (3a) est un anticorps.

2. Procédé selon la revendication 1, comprenant en outre une étape de lavage du substrat (4) avec une solution de lavage avant l'étape d).

3. Procédé selon la revendication 1 ou 2, dans lequel la solution de lavage comprend un détergent à un niveau de concentration inférieur à 5 %, de préférence inférieur à 1 %, plus préférablement, inférieur à 0,5 %, dans lequel le détergent est de préférence non ionique, notamment un monolaurate de polyoxyéthylène sorbitan.

4. Procédé selon l'une quelconque des revendications précédentes, dans lequel l'échantillon est mis en contact avec le réactif de détection (3) pendant une durée inférieure à 24 heures avant l'étape c), de préférence inférieure à 2 heures avant l'étape c), plus préférablement inférieure à 20 minutes avant l'étape c), plus préférablement encore inférieure à 10 minutes avant l'étape c), et de manière préférée entre toutes inférieure à 1 minute avant l'étape c).

5. Procédé selon l'une quelconque des revendications précédentes, dans lequel le fragment de capture (1b) est la biotine et le substrat (4) ayant une surface comportant des sites réactifs comprend de la streptavidine.

6. Procédé selon l'une quelconque des revendications précédentes, dans lequel le fragment détectable (3b) est choisi dans un groupe de réactifs constitué par : Alexa Fluor 633, Alexa Fluor 647, DyLight 649, Cy-5, Cy-5.5, et les nanoparticules fluorescentes.

7. Procédé selon l'une quelconque des revendications précédentes, dans lequel l'échantillon a un volume inférieur à, ou égal à, 1000 nl, de préférence inférieur à, ou égal à, 200 nl.

8. Procédé selon l'une quelconque des revendications précédentes, dans lequel le réactif de détection (3) a un volume inférieur à, ou égal à, 1000 nl, de préférence inférieur à, ou égal à, 200 nl.

9. Procédé selon l'une quelconque des revendications précédentes, dans lequel le réactif de détection (3) a une concentration inférieure à 15 µg/ml, ou inférieure à 100 nM, de préférence inférieure à 0,5 µg/ml, ou inférieure à 3,3 nM.

10. Procédé selon l'une quelconque des revendications précédentes, dans lequel le substrat (4) comprend des particules qui sont poreuses, ou semi-pelliculaires, ou pelliculaires (i.e ;, non poreuses), dans lequel les particules sont de préférence essentiellement circulaires et ont un diamètre moyen supérieur à 10 µm, et/ou dans lequel les particules ont de préférence une distribution de taille uniforme.

11. Procédé selon l'une quelconque des revendications précédentes, dans lequel le substrat (4) fait partie d'un dispositif, le dispositif comprenant :
- une admission pour l'échantillon ; et
- une admission pour le réactif de détection ; et
- une admission pour le réactif de capture ; et
- une chambre de mélange en contact fluidique avec l'admission pour l'échantillon et l'admission pour le réactif de détection, la chambre de mélange se trouvant dans une direction en aval de l'admission pour l'échantillon et de l'admission pour le réactif de détection ; et
- une colonne comprenant le substrat (4), la colonne étant en contact fluidique avec l'admission pour l'échantillon, l'admission pour le réactif de détection, l'admission pour le réactif de capture, et la chambre de mélange, et la colonne se trouvant dans une direction en aval de l'admission pour le réactif de capture et de la chambre de mélange ; et
- une évacuation en contact fluidique avec la colonne, l'évacuation se trouvant dans une direction en aval de la colonne ; et
- un moyen pour détecter le réactif de détection.
